# EUROPÄISCHE PATENTSCHRIFT

(11) **EP 3 271 374 B1**
(45) Veröffentlichungstag und Bekanntmachung des Hinweises auf die Patenterteilung: **03.06.2020**
(21) Anmeldenummer: 16716456.5
(22) Anmeldetag: 25.02.2016
(51) Int. Cl.: C07K 7/08

(54) **SPEZIFISCH A-BETA-SPEZIES BINDENDE PEPTIDE FÜR DIE THERAPIE UND/ODER DIE DIAGNOSE DER ALZHEIMERSCHEN DEMENZ**
PEPTIDES WHICH BIND TO A SPECIFIC A-BETA-SPECIES FOR THE THERAPY AND/OR DIAGNOSIS OF ALZHEIMER'S DISEASE
PEPTIDES SE LIANT SPÉCIFIQUEMENT À DES ESPÈCES A-BÊTA POUR LA THÉRAPIE ET/OU LE DIAGNOSTIC DE LA DÉMENCE D'ALZHEIMER

(30) Priorität: 20.03.2015 DE 102015003676
(43) Veröffentlichungstag der Anmeldung: 24.01.2018
(73) Patentinhaber: Forschungszentrum Jülich GmbH, 52425 Jülich (DE)
(72) Erfinder: WILLBOLD, Dieter, 52428 Jülich (DE); KUTZSCHE, Janine, 52353 Düren (DE); KLEIN, Antonia, Nicole, 31275 Lehrte-Arpke (DE)
(74) Vertreter: Fitzner, Uwe
(86) Internationale Anmeldenummer: PCT/DE2016/000089
(87) Internationale Veröffentlichungsnummer: WO 2016/150415

(56) Entgegenhaltungen:
- WO-A1-2015/043566
- WO-A2-02/081505
- WO-A2-2013/150126
- WO-A2-2014/041115
- SCHUMACHER T N M ET AL: "IDENTIFICATION OF D-PEPTIDE LIGANDS THROUGH MIRROR-IMAGE PHAGE DISPLAY", SCIENCE, AMERICAN ASSOCIATION FOR THE ADVANCEMENT OF SCIENCE, US, Bd. 271, 29. März 1996 (1996-03-29), Seiten 1854-1857, XP000650829, ISSN: 0036-8075, DOI: 10.1126/SCIENCE.271.5257.1854

## Beschreibung

Die Erfindung bezieht sich auf spezifisch A-Beta-Spezies bindende Peptide für die Therapie und/oder die Diagnose der Alzheimerschen Demenz.

### Stand der Technik

Noch immer existiert kein zugelassenes Medikament für eine ursächliche Behandlung der Alzheimerschen Demenz (AD). Typischerweise findet man in den Gehirnen von AD-Patienten post-mortem Ablagerungen des sogenannten Beta-Amyloid-Peptides (Aß) in Plaques. Deshalb werden schon lange verschiedene Formen des A-Beta (auch Aß genannt), z.B. Fibrillen, für die Entstehung und das Fortschreiten der Alzheimerschen Demenz verantwortlich gemacht.

Seit wenigen Jahren werden besonders die kleinen, frei diffundierbaren Aß-Oligomere als hauptsächliche Verursacher für die Entstehung und den Fortschritt der Alzheimerschen Demenz verantwortlich gemacht.

Aß-Monomere entstehen ständig in unserem Körper und sind vermutlich per se nicht toxisch.

Es wird spekuliert, ob sich Aß-Monomere abhängig von ihrer Konzentration, die sich letztlich durch Bildungs- und Abbau-Raten im Körper ergibt, zufällig und damit mit zunehmendem Alter immer wahrscheinlicher spontan zu Aβ-Oligomeren zusammen lagern. Einmal entstandene Aβ-Oligomere könnten sich dann durch einen Prionähnlichen Mechanismus vermehren und letztlich zur Krankheit führen.

Aufgrund dieser Überlegungen sollte es das Ziel einer ursächlichen Behandlung sein, toxische Aβ-Oligomere vollständig zu vernichten und/oder deren Prion-ähnliche Vermehrung zu verhindern.

Allerdings existiert kein ursächlich wirkendes Medikament für die Behandlung der Alzheimerschen Demenz. Die eingesetzten Medikamente nach dem Stand der Technik sind bestenfalls in der Lage, einige Symptome zu mildern, können aber den Krankheitsfortschritt nicht verlangsamen, geschweige denn aufhalten.

Es existieren eine Reihe von Substanzen, die in der Lage sind, im Tierversuch einige Erfolge bei der Prävention (nicht unbedingt die Behandlung) der Alzheimerschen Demenz zu erreichen.

Ein wichtiger Unterschied zwischen der Prävention und der Behandlung liegt in folgender Überlegung: Um die Bildung der ersten Aβ-Oligomere zu verhindern, reichen eventuell schon wenig affine und effektive Aß-Liganden aus. Da die Bildung eines Aβ-Oligomers aus mehreren Aß-Monomeren eine Reaktion sehr hoher Ordnung ist, ist sie in hoher Potenz von der Aß-Monomer-Konzentration abhängig. Auch eine kleine Verringerung der aktiven Aß-Monomer-Konzentration kann so die Bildung der ersten Aβ-Oligomere verhindern. Dies ist die Situation bei der Prävention. Sind aber bereits Aβ-Oligomere entstanden, können sich diese Prion ähnlich vermehren, was keine Reaktion hoher Ordnung ist und damit kaum noch abhängig von der Aß-Monomer-Konzentration ist. Dies ist die Situation bei der Therapie. Sind also Aß-Oligomere bereits entstanden, muss es das Ziel einer Behandlung sein, diese durch Substanzen zu adressieren, die eine möglichst hohe Affinität zu Aβ-Oligomeren besitzen. Die entsprechende Dissoziationskonstante müsste dazu im pM-Bereich oder noch tiefer liegen.

Derzeit existieren einige Substanzen, die auf verschiedenste Art und Weise die Konzentration von Aß-Monomeren verringern, z. B. durch gamma-Sekretase-Modulatoren, Aß-bindende Liganden, und so weiter. Dies reicht offensichtlich aus, um im Tierversuch präventiv erfolgreich zu wirken, wobei meist Tiere behandelt werden, bevor die Krankheit voll ausbricht.

In klinischen Studien (Phasen II und III) am Menschen, hier dürfen nur klar mit der Alzheimerschen Demenz diagnostizierte Menschen behandelt werden, versagten bisher aber alle diese Substanzen, möglicherweise, weil hier also noch vor Beginn der Erkrankung eine geringe oder mäßige Verringerung der Aß-Monomer-Konzentration nicht mehr ausreicht, um die Entstehung immer größerer Mengen von Aβ-Oligomeren zu verhindern.

Desweiteren gibt es bisher keine Möglichkeit, die Alzheimersche Demenz vor dem Ausbruch der Symptome sicher zu diagnostizieren. Die Alzheimersche Demenz wird heute hauptsächlich durch neuropsychologische Tests der bereits an Demenzsymptomen leidenden Person erkannt. Des Weiteren können andere Erkrankungen (Traumata) durch verschiedene Untersuchungsmethoden ausgeschlossen werden.

Es ist jedoch bekannt, dass Aβ-Oligomere und zeitlich darauf folgend Plaques bis zu 20 Jahre vor dem Auftreten der Symptome im Gehirn der Patienten entstehen und irreversiblen Schaden anrichten. Molekulare Sonden, die dem Patienten intravenös injiziert werden und die nach der Passage über die Blut-Hirn-Schranke an Aß-Oligomere und Plaques binden, können mittels bildgebender Methoden sichtbar gemacht werden und somit eine früher Diagnose der AD ermöglichen.

Die WO 02/081505 A2 betrifft ein Peptid, das mit hoher Bindungsaffinität an das β-Amyloid-Peptid bindet. Die WO 2013/150126 A2 betrifft ein Verfahren zur Behandlung von Blut, Blutprodukten und Organen zur Prävention der Übertragung von Morbus-Alzheimer. Die WO 2015/043566 A1 bezieht sich auf zyklische, Amyloid-Betabindende Peptide und deren Verwendung. Schumacher T N et al. ("Identification of D-peptide ligands through mirror-image phage display", Science, Bd. 271, 29. März 1996, Seiten 1854-1857) beschreiben D-Peptidliganden mit Resistenz gegenüber proteolytischem Abbau.

Aus EP 1 379 546 B1 ist ein D-enantiomeres Peptid mit dem Namen "D3" bekannt. Das Peptid wurde durch eine Spiegelbild-Phagendisplay-Selektion gegen überwiegend monomeres Aß(1-42) identifiziert, mit dem Plan, dieses durch die Bindung zu stabilisieren und seine Umwandlung in toxische Aß-Aggregate zu verhindern. Nach derzeitigem Kenntnisstand wandelt D3 bevorzugt die besonders toxischen Aß-Oligomere in nicht-toxische, nicht-amyloidogene und ThT-negative, amorphe Aggregate um. Im Tiermodell wird selbst durch eine orale Verabreichung von D3 mit dem Trinkwasser erreicht, dass behandelte transgene AD-Mäuse deutlich weniger Plaques enthalten und signifikant verbesserte kognitive Fähigkeiten besitzen.

Aus der Veröffentlichungsschrift WO 2014/041115 A2 sind weitere von D3 abgeleitete Peptide bekannt.

Nachteilig sind die bestehenden Aβ-Oligomer-bindenden Substanzen nicht genügend affin, um eine Vermehrung von Aβ-Oligomeren zu verhindern.

Es gibt nachteilig bisher keine Sonden für das in-vivo-Imaging-Verfahren, die speziell an Aβ-Oligomere hochaffin binden und diese sichtbar machen. Da Aβ-Oligomere in der Krankheitsgeschichte eine so wichtige und frühe Rolle spielen, ist genau dieses wünschenswert.

### Aufgabe der Erfindung

Aufgabe der Erfindung ist es Peptide bereit zu stellen, für die
A) Ursächliche Therapie von Morbus Alzheimer durch das Verhindern der Bildung von toxischen Amyloid-β (Aβ)-Oligomeren oder Aggregaten oder deren Enttoxifizierung.
B) Diagnose der Alzheimerschen Demenz durch Anwendung der D-Peptide als Sonde für in vivo Imaging.
Aufgabe war es außerdem neue Peptide zur Verfügung zu stellen, bevorzugt Derivate des D-enantiomeren D-Peptids D3, die im Vergleich zu D3 effizientere Eigenschaften besitzen. Die Eigenschaften umfassen unter anderem Bindungsaffinität und -spezifität für A-Beta-Spezies, Inhibition der A-Beta-Fibrillenbildung, Inhibierung der A-Beta-Zytotoxizität, Eliminierung oder Enttoxifizierung von A-Beta-Oligomeren, Fibrillen und anderen Aggregaten, Umwandlung von A-Beta-Amyloid-Fibrillen, - Protofibrillen, oder -Oligomere in nicht-toxische, nicht-amyloidogene Spezies.

Im Nachfolgenden werden die Begriffe "A-Beta", "Amyloid-Beta", "Amyloid-β" bzw. "Aβ" synonym zueinander gebraucht.

### Lösung der Aufgabe

Die Aufgabe der Erfindung wird gelöst durch die Peptide nach Patentanspruch 1, dem Kit und der Zusammensetzung gemäß der Hauptansprüche sowie den Verwendungen gemäß den Nebenansprüchen. Vorteilhafte Ausgestaltungen hierzu ergeben sich jeweils aus den hierauf rückbezogenen Patentansprüchen.

### Beschreibung der Erfindung

Gelöst wird diese Aufgabe durch spezifisch Amyloid-Beta-Spezies bindende Peptide für die Therapie der Alzheimerschen Demenz. Die Spezifität, oft auch Selektivität genannt, bedeutet dabei, dass der erfindungsgemäße Ligand bzw. ein erfindungsgemäßes Peptid grundsätzlich verschiedene A-Beta-Spezies, z.B. A-Beta-Monomer oder A-Beta-Oligomer oder A-Beta-Fibrillen binden kann. Jedem Bindungspaar (z. B. Ligand zu A-Beta-Monomer und Ligand zu A-Beta-Oligomer und Ligand zu A-Beta-Fibrillen) wird unter der Annahme eines 1:1-Bindemodells jeweils eine Dissoziationskonstante (K_{D}) oder eine Assoziationskonstane (K_{A}=1/K_{D}) zugeordnet. Je höher der K_{A}-Wert ist, desto affiner ist die jeweilige Bindung.

Die Spezifität des Liganden für den Köder gegenüber dem Kompetitor drückt sich durch einen möglichst großen Quotienten der Affinitäten zum A-Beta-Monomer und zum A-Beta-Oligomer bzw. A-Beta-Fibrillen aus.

Erfindungsgemäß werden zur Ermittlung der Spezifität also Quotienten der K_{A}-Werte des Liganden zum A-Beta-Monomer und zum A-Beta-Oligomer (bzw. A-Beta-Fibrillen) gebildet. Je größer der Quotient ist, umso größer ist die Spezifität.

In erfindungsgemäßen Sinn ist von relativer Spezifität die Rede, wenn der Quotient der K_{A}-Werte des Liganden zur Spezies A und des Liganden zur Spezies B mindestens größer als 1 ist, bevorzugt größer als 1,2, oder 1,5, bevorzugt größer als 2, 3, 4, 5, 6, 7, 8, 9, insbesondere 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, insbesondere 100, wobei als Spezies A und Spezies B die verschiedenen A-Beta-Spezies Monomer, Oligomer und Fibrillen angenommen werden können.

Die Spezifität oder Selektivität ist dabei nicht mit der Affinität zu verwechseln, die sich aus der Bindungsstärke von Liganden an eine der Spezies ergibt. Konkurrieren verschiedene Liganden um das gleiche Zielmolekül, so kann der weniger spezifische oder selektive die größere Bindungsaffinität besitzen. Erfindungsgemäß soll derjenige Ligand ermittelt werden, welcher besonders spezifisch an eine Spezies wie Monomer oder Oligomer oder Fibrillen bindet. Die Biomoleküle sollen als therapeutische Mittel und Heilmittel geeignet sein. Die auf diese Weise erhaltenen Liganden bzw. Peptide haben die gewünschten Eigenschaften und lösen die Aufgabe der Erfindung.

Insbesondere lösen Peptide als Liganden enthaltend eine Aminosäuresequenz gemäß der SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 und SEQ ID NO: 11 und deren Homologe mit einer Identität der Aminosäuresequenz von mindestens 80%, wobei die Homologe einen Kd-Wert von 2-fach bis 10-fach niedriger als das Peptid mit der Aminosäuresequenz rprtrlhthrnr aufweisen, wobei das Peptid im Wesentlichen aus D-Aminosäuren besteht, die Aufgabe der Erfindung.

Gelöst wird diese Aufgabe auch durch Polymere enthaltend eine Aminosäuresequenz gemäß der SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 oder SEQ ID NO: 11 und/oder deren Homologe mit einer Identität der Aminosäuresequenz von mindestens 80%, wobei die Homologe einen Kd-Wert von 2-fach bis 10-fach niedriger als das Peptid mit der Aminosäuresequenz rprtrlhthrnr aufweisen, wobei das Peptid im Wesentlichen aus D-Aminosäuren besteht.

Die Peptide enthaltend eine der genannten Sequenzen binden spezifisch an Amyloid-Beta-Monomer.

Spezifische Bindung zu A-Beta-Monomer bedeutet im Übrigen, dass der Quotient der Bindungssignale von Ligand-Monomer zu Ligand-Oligomer und/oder Ligand-Monomer zu Ligand-Fibrillen größer ist als 1, z. B. 1,8 und etwa einen Wert bis 100 annehmen kann, wobei jeder Zwischenwert z. B. 2, 3, 4, 5, 6, 7, 8, 9, insbesondere 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, insbesondere 100 angenommen werden kann.

Spezifische Bindung zu A-Beta-Oligomer bedeutet, dass der Quotient der Bindungssignale aus Ligand-Oligomer zu Ligand-Monomer bzw. aus Ligand-Oligomer zu Ligand-Fibrillen größer ist als 1, z. B. 1.2, und etwa einen Wert bis 100 annehmen kann, wobei jeder Zwischenwert z. B. 2, 3, 4, 5, 6, 7, 8, 9, insbesondere 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, insbesondere 100 angenommen werden kann.

Die in der wissenschaftlichen Praxis üblichen Rundungen ungerader Zahlenwerte führen zu den oben genannten Zahlenwerten.

Als Ausgangspunkt dienen z. B. durch Peptide Microarray Experimente erhaltene Peptide. Diese können von dem bekannten Peptid "D3" abgeleitet werden.

Für die Identifizierung von spezifisch an Amyloid-Beta-Monomer oder spezifisch an Amyloid-Beta-Oligomere bindenden Peptiden und Bestimmung der Bindungsspezifität von Peptiden im Sinne der vorliegenden Erfindung kann demnach man wie folgt vorgehen. Peptide Microarrays mit verschiedenen auf einer Oberfläche immobilisierten Peptiden, z. B. Derivate von D3, werden jeweils mit verschiedenen Spezies von A-Beta (z.B. Monomere, Oligomere und Fibrillen), welches mit einem Fluoreszenzfarbstoff gelabelt ist, inkubiert. Als Kontrolle wird ein Peptid Microarray mit nur dem Fluoreszenzfarbstoff inkubiert. Um die Reproduzierbarkeit der Ergebnisse zu gewährleisten, kann die Inkubation der Peptide Microarrays je Spezies mindestens 3 x durchgeführt werden. Die Bindung des Fluoreszenz gelabelten A-Beta mit den immobilisierten Peptiden wird über den Fluoreszenzfarbstoff detektiert. Je mehr A-Beta an dem Peptid bindet, umso höher ist das Fluoreszenzsignal. Die gemessenen Fluoreszenzsignale der Reproduktionen werden normiert und das Signal von der Kontrolle abgezogen. Damit wird verhindert, dass die Bindung des Fluoreszenz gelabelten A-Beta über den Farbstoff stattfindet. Sodann werden die Bindungssignale von einer A-Beta Spezies an ein Peptid gemittelt. Für die Bestimmung der Bindungsspezifität wird der Quotient von der Spezies, zu dem das Peptide spezifisch binden soll, zu einer anderen Spezies gebildet.

Beispiel: Bindungsspezifität des Peptides X zu A-Beta-Monomeren = Bindungssignal von Peptid X an Monomere/Bindungssignal von Peptid X an Oligomere (oder Fibrillen). In analoger Weise werden spezifisch an A-Beta-Oligomer bindende Peptide ermittelt.

Fragmente und Teile der erfindungsgemäßen Peptide zeigen eine ähnliche beziehungsweise identische Wirkung wie die erfindungsgemäßen Peptide.

In einer Variante der Erfindung werden solche Peptide ermittelt und eingesetzt, die an ein A-Beta-Monomer oder A-Beta-Oligomer oder A-Beta-Fibrillen mit einem K_{D} von höchstens 500 µM, bevorzugt 250, 100, 50 µM, besonders bevorzugt 25, 10, 6 µM, insbesondere 4, 2, 1 µM oder sub-µM bindet.

Die Peptide gemäß der SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO. 10, und/oder SEQ ID NO. 11 und deren Homologe bestehen im Wesentlichen, bevorzugt mindestens 60%, 75%, 80%, besonders bevorzugt 85%, 90%, 95%, insbesondere 96%, 97%, 98%, 99%, 100% aus D-Aminosäuren.

Ein Polymer im Sinne der Erfindung ist gebildet aus mindestens 2, oder 3, 4, 5, 6, 7, 8, 9, 10, 11, 12, 13, 14, 15, 16, 17, 18, 19, 20 oder mehr Monomeren oder Monomereinheiten ausgewählt aus der Gruppe bestehend aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO. 10, und/oder SEQ ID NO. 11 und deren Homologe, Fragmente oder Teile, die für sich bereits A-Beta-Monomere oder A-Beta-Oligomere oder A-Beta-Fibrillen binden.

Erfindungsgemäß sind die Polymere aus identischen Monomereinheiten aufgebaut oder aus einer Kombination von 2, 3, 4, 5, 6, 7, 8, 9, 10 oder 11 unterschiedlichen, verschiedenen der oben genannten Monomeren, als sogenannte Kombinations-Polymere. Die Monomere können auch teilweise identisch sein. Die Anzahl der identischen Monomeren in den Kombinations-Polymeren ist frei wählbar.

In einer Alternative enthalten die Kombinations-Polymere mindestens ein Monomer ausgewählt aus der Gruppe bestehend aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO. 10 und/oder SEQ ID NO. 11 und deren Homologe und mindestens ein an A-Beta-Monomere oder an A-Beta-Oligomere oder A-Beta-Fibrillen bindendes Peptid, das sich von den Monomeren ausgewählt aus der Gruppe bestehend aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO. 10 und/oder SEQ ID NO. 11 und deren Homologe unterscheidet, als weiteres Monomer.

Polymere können beispielsweise über chemische Synthese bzw. Peptidsynthese hergestellt werden.

In einer Ausführung der Erfindung sind die Monomere kovalent miteinander verknüpft. In einer anderen Ausführung der Erfindung sind die Monomere nicht kovalent miteinander verbunden.

Eine kovalente Verbindung bzw. Verknüpfung der Monomer-Einheiten liegt im Sinne der Erfindung vor, falls die Peptide Kopf an Kopf, Schwanz an Schwanz oder Kopf an Schwanz linear miteinander verknüpft werden, ohne dass dazwischen Linker oder Linker-Gruppen eingesetzt werden.

Eine nicht kovalente Verknüpfung im Sinne der Erfindung liegt vor, falls die Monomere über Biotin und Streptavidin, insbesondere Streptavidin-Tetramer miteinander verknüpft sind.

In einer Variante der vorliegenden Erfindung können die Monomere linear miteinander verknüpft sein, insbesondere wie oben beschrieben. In einer anderen Variante sind die Monomere verzweigt miteinander zu dem erfindungsgemäßen Polymer verknüpft.

Bei einem verzweigten Polymer kann es sich erfindungsgemäß um ein Dendrimer handeln, bei dem die Monomere kovalent oder nicht kovalent miteinander verknüpft sind.

Alternativ können die Monomere auch mit einem Plattform-Molekül (wie z.B. PEG oder Zucker) verknüpft sein und so ein verzweigtes Polymer bilden.

Alternativ sind auch Kombinationen dieser Optionen möglich.

Die erfindungsgemäß aufgebauten Polymere aus erfindungsgemäß Peptiden, die ihrerseits an A-Beta-Monomer oder Abeta-Oligomer oder A-Beta-Fibrillen binden, zeigen vorteilhaft synergetische Effekte in Bezug auf ihre Selektivität und Affinität zu den A-Beta-Monomer zu einzelnen Peptiden. Mit anderen Worten: Die erfindungsgemäßen Polymere sind den Einzelpeptiden, aus denen sie aufgebaut sind, überlegen. Synergetische Effekte im Sinne der vorliegenden Erfindung sind Effekte, die eine höhere Selektivität bzw. Spezifität und / oder Affinität bezüglich des A-Beta-Monomer oder A-Beta-Oligomers oder A-Beta-Fibrillen zeigen, insbesondere des K_{D}-Wertes betreffend die Bindung an A-Beta-Monomer oder A-Beta-Oligomer oder A-Beta-Fibrillen im Vergleich zu den einzelnen Peptid-Einheiten.

In einer weiteren besonders vorteilhaften Ausgestaltung der Erfindung wirken die Polymere und insbesondere Dimere im Tiermodellversuch (in vitro bzw. in vivo) vorteilhaft effizienter als die einzelnen Peptid-Einheiten.

In einer Variante der Erfindung werden solche Peptide oder Polymere eingesetzt, die an ein A-Beta-Monomer oder A-Beta-Oligomer oder A-Beta-Fibrillen mit einer Dissoziationskonstante (K_{D}-Wert) von höchstens 500 µM, bevorzugt 250, 100, 50 µM, besonders bevorzugt 25, 10, 1 µM, besonders bevorzugt mit einer Dissoziationskonstante (K_{D}-Wert) von höchstens 500 nM, 250, 100, 50, besonders bevorzugt 25, 10, 1 nM, 500pM, 100, 50, 25, 20, 15, 10, 9, 8, 7, 6, 5, 4, 3, 2, 1 pM bis sub-pM bindet, wobei jeder Zwischenwert angenommen werden kann.

In einer Ausgestaltung der Erfindung ist die Affinität über die Dissoziationskonstante (K_{D}-Wert) definiert. Die Dissoziationskonstante (K_{D}-Wert) des erfindungsgemäßen Peptids ist dabei in einer Ausgestaltung der Erfindung erniedrigt. Damit ist eine höhere Affinität der Bindung und eine höhere Effektivität des Abbaus und / oder der Verhinderung der Bildung toxischer Amyloid-Beta-Oligomere. Dies betrifft insbesondere aber nicht ausschließlich einen niedrigeren K_{D}-Wert an der high affinity site des A-Beta-Monomer oder A-Beta-Oligomer.

Fragmente und Teile zeigen vorteilhaft eine ähnliche beziehungsweise identische Wirkung wie die erfindungsgemäßen Peptide.

Monomere und Polymere werden im Folgenden als erfindungsgemäße Peptide bezeichnet.

Die erfindungsgemäßen Peptide sind in einer Ausgestaltung der Erfindung am freien C-terminus mit einer Säureamidgruppe versehen. Die erfindungsgemäßen Peptide, beispielweise die Peptide gemäß SEQ ID NO: 1-11 sind dann an der Position 12 am freien C-terminus amidiert. Dimere hieraus sind an Position 24 am freien C-terminus amidiert und so weiter.

Die erfindungsgemäßen Peptide, insbesondere die Peptide gemäß der SEQ ID NO: 1-11 sind in einer weiteren Ausgestaltung der Erfindung am freien C-terminus mit dem freien N-terminus kovalent miteinander verbunden und liegen dann entsprechend zyklisiert vor. Auch durch den Ringschluss wird vorteilhaft bewirkt, dass die Carboxylgruppe am freien C-terminus nicht mehr vorhanden ist.

Das erfindungsgemäße Peptid weist vorteilhaft eine Aminosäuresequenz auf, bei der die Zyklisierung des linearen Moleküls z. B. durch eine kovalente Bindung der ersten mit der letzten Aminosäure, z.B. über eine Kondensationsreaktion, erfolgt ist. Selbstverständlich existieren weitere Möglichkeiten der Zyklisierung, z. B. indem andere Aminosäuren miteinander verknüpft werden. Lediglich beispielhaft sei die Verknüpfung der zweiten Aminosäure mit der letzten Aminosäure genannt. Genauso denkbar ist jede mögliche andere Verknüpfung.

Im Falle, dass die erste und die letzte Aminosäure des Peptids miteinander verknüpft werden, wird vorteilhaft bewirkt, dass keine offenen Enden in der Peptidkette (Aminosäuresequenz) vorliegen.

Diese Maßnahme bewirkt ferner, dass alle Peptide mit linearen Aminosäuresequenzen, die nach der Zyklisierung die gleiche, nicht mehr unterscheidbare Aminosäure-Reihenfolge ergeben, in diesem Sinne identisch sind.

Beispiel: Die lineare Aminosäuresequenz des bekannten Peptids D3 ist rprtrlhthrnr. Das entsprechende durch eine Amidbindung zwischen der N-terminalen Aminogruppe und der C-terminalen Carboxylgruppe verknüpfte, zyklisierte Peptid "cD3" ist nicht mehr zu unterscheiden von den zyklisierten Peptiden prtrlhthrnrr, rtrlhthrnrrp, trlhthrnrrpr, rlhthrnrrprt, Ihthrnrrprtr, hthrnrrprtrl, thrnrrprtrlh, hrnrrprtrlht, rnrrprtrlhth, nrrprtrlhthr, oder rrprtrlhthrn. Aus jeder dieser Sequenzen lässt sich das cD3 weiterhin auch ableiten.

Die erfindungsgemäß beanspruchten Effekte der höheren Affinität, Spezifität und Effektivität treten darüber hinaus gegenüber einem, vorzugsweise sogar jedem linearen, bindenden Peptid auf, aus dem sich ein zyklisiertes bzw. anderweitig modifiziertes, erfindungsgemäßes Peptid ableiten lässt.

Die Herstellung zyklisierter Peptide ist im Übrigen Stand der Technik, und kann beispielweise nach dem Verfahren wie es in DE 102005049537 A1 beschrieben ist, erfolgen.

Vorteilhaft bewirkt die Zyklisierung über die erste und letzte Aminosäure des Peptids, dass keine "offenen" Enden der Peptidkette mehr existieren, die oft Angriffspunkte für Peptid-abbauende Aktivitäten in Zellen, Tieren oder Menschen sind, z. B. durch Aminopeptidasen und Carboxypeptidasen.

Mittels erfindungsgemäßer zyklisierter Peptide, wie z. B. ANK6 gemäß der SEQ ID NO: 1, wird zusätzlich vorteilhaft bewirkt, dass diese erfindungsgemäßen zyklisierten Peptide, bzw. Polymere als Seiteneffekt unter Umständen auch nicht leicht abgebaut werden, wenngleich dieser Effekt nicht ausschlaggebend ist. Er gilt im Übrigen, wie gezeigt wurde, auch nur für den Fall einer head-to-tail- oder tail-to-head-Zyklisierung, bei dem entsprechend beide Enden des linearen Peptids miteinander verknüpft werden.

"Homologe Sequenzen" oder "Homologe" bedeutet im Sinne der Erfindung, dass eine Aminosäuresequenz eine Identität mit einer der oben genannten Aminosäuresequenz der Monomere von mindestens 80, 81, 82, 83, 84, 85, 86, 87, 88, 89, 90, 91, 92, 93, 94, 95, 96, 97, 98, 99, aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz/ Polypeptidsequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3.

Zwei Aminosäuresequenzen sind im Sinne der vorliegenden Erfindung identisch wenn sie dieselbe Aminosäuresequenz besitzen.

Unter Homologe sind in einer Variante die entsprechenden retro-inversen Sequenzen der oben genannten Monomere zu verstehen. Mit dem Begriff "retro-inverse Sequenz" wird erfindungsgemäß eine Aminosäuresequenz bezeichnet, die sich aus Aminosäuren in der enantiomeren Form zusammensetzt (invers: Chiralität des alpha-C-Atoms invertiert) und bei der zusätzlich die Sequenzreihenfolge zur ursprünglichen Aminosäuresequenz umgekehrt wurde (retro = rückwärts).

In einer weiteren Variante binden die erfindungsgemäßen Peptide an Teile des Amyloid Beta-Peptids.

In einer weiteren Variante weisen die erfindungsgemäßen Peptide Sequenzen auf, die sich von den angegebenen Sequenzen um bis zu drei Aminosäuren unterscheiden.

Ferner werden als Peptide auch Sequenzen eingesetzt, die die oben genannten Sequenzen enthalten.

In einer weiteren Variante weisen die Peptide Fragmente der oben genannten Sequenzen auf oder weisen homologe Sequenzen zu den oben genannten Sequenzen auf.

Erfindungsgemäß handelt es sich um ein Peptid zur Verwendung in der Medizin, bevorzugt zur Behandlung oder Diagnose von Morbus Alzheimer.

Das Peptid besteht im Wesentlichen aus D-Aminosäuren.

Im Sinne der vorliegenden Erfindung bedeutet der Begriff "im Wesentlichen aus D-Aminosäuren", dass die erfindungsgemäß einzusetzenden Monomere mindestens 50%, 55%, 60%, 65%, 70%, bevorzugt 75%, 80%, besonders bevorzugt 85%, 90%, 95%, insbesondere 96%, 97%, 98%, 99%, 100% aus D-Aminosäuren aufgebaut sind.

In einer Ausführung der vorliegenden Erfindung handelt es sich bei den erfindungsgemäßen Peptiden um Derivate des D-enantiomeren D-Peptids D3. Derivate im Sinne der Erfindung sind von D3 abgeleitete Peptid-Sequenzen, die nach einem der drei folgenden Verfahren erzielt werden:
a) Änderung der Reihenfolge und/oder Anzahl der Aminosäurebausteine in D3. Es werden dabei nur Aminosäuren eingesetzt, die in der D3-Sequenz vorhanden sind.
b) Deletion von 1, 2, 3, 4, 5, 6, 7, 8, 9, 10 oder 11 Aminosäuren der D3-Sequenz.
c) Austausch von 1, 2, 3, 4, 5 oder 6 Aminosäuren durch andere Aminosäuren, bevorzugt D-Enantiomere.

In einer weiteren Variante handelt es sich um ein erfindungsgemäßes Peptid zur Hemmung der Fibrillenbildung von Amyloid-Beta-Peptiden. Die erfindungsgemäßen Peptide und/oder Polymere enttoxifizieren die A-Beta-Oligomere oder daraus gebildete Polymere, sowie Fibrillen, indem sie z. B. an A-Beta-Monomere binden und so in nicht toxische Verbindungen überführen. Demgemäß ist Gegenstand der vorliegenden Erfindung auch ein in-vitro Verfahren zur Enttoxifizierung von A-Beta-Oligomeren, sowie der daraus gebildeten Polymere oder Fibrillen.

Gegenstand der Erfindung sind in einer Ausführung auch erfindungsgemäße Peptide, die mit einer weiteren Substanz verknüpft sind.

Bei der Verknüpfung handelt es sich im Sinne der Erfindung um eine chemische Bindung wie sie in Römpp Chemie Lexikon, 9. Auflage, Band 1, Seite 650 ff, Georg Thieme Verlag Stuttgart definiert ist, bevorzugt um eine Hauptvalenz Bindung, insbesondere eine kovalente Bindung.

Bei den Substanzen handelt es sich in einer Variante um Arzneimittel oder Wirkstoffe, definiert gemäß Arzneimittelgesetz §2 beziehungsweise. §4 (19), Stand September 2012. In einer Alternative sind Wirkstoffe therapeutisch aktive Stoffe die als arzneilich wirksame Stoffe verwendet werden. Bevorzugt werden Entzündungshemmer eingesetzt.

Bei den Substanzen handelt es sich in einer weiteren Variante um Verbindungen die die Wirkung der Peptide verstärken.

In einer Alternative sind solche Verbindungen Aminopyrazol und/oder Aminopyrazolderivate. Aminopyrazolderivate im Sinne der Erfindung ist 3-Aminopyrazol-5-carbonsäure oder 3-Nitropyrazol-5-carbonsäure sowie alle Abkömmlinge, in denen die heterocyclische CH-Gruppe gegen -CR- oder -N- oder -O- oder -S- ausgetauscht wurde, sowie alle daraus abgeleiteten peptidischen Dimere, Trimere oder-Tetramere, bevorzugt Aminopyrazol-Trimer.

In einer weiteren Alternative handelt es sich um Verbindungen die die Löslichkeit der Peptide und/oder die Passage der Blut-Hirn-Schranke verbessern.

In einer Alternative haben die Peptide erfindungsgemäß jede beliebige Kombination von mindestens zwei oder mehr Merkmalen der oben beschriebenen Varianten, Ausführungen und/oder Alternativen.

Im Rahmen der Erfindung wurde weiterhin erkannt, dass amidierte und/oder zyklisierte Peptide im Vergleich zu linearen, bindenden Peptiden, bei denen der freie C-terminus, also die C-terminale Carboxylguppe, nicht modifiziert ist und entsprechend eine negative Ladung trägt, mit einer höheren Affinität an Abeta-Monomer binden. Das heißt, dass der Ko-Wert bei den modifizierten Peptiden niedriger ist, als bei linearen Peptiden, bei denen der freie C-terminus, also die C-terminale Carboxylguppe, nicht modifiziert ist und entsprechend eine negative Ladung trägt.

In einer weiteren bevorzugten Ausgestaltung der Erfindung ist daher die Affinität der Bindung der erfindungsgemäß modifizierten Peptide ohne negative Ladung am C-terminus im Vergleich zu linearen Peptiden mit negativer Ladung am C-terminus aber im Übrigen gleicher Aminosäuresequenz, um 1%, 2, 3, 4, 5, 6, 7, 8, 9, insbesondere 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, insbesondere 100%, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, insbesondere 200 %, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, insbesondere 300%, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, insbesondere 400%, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, vorteilhaft sogar 500%, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, besonders vorteilhaft 600%, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, besonders vorteilhaft 700%, 701, 702, 703, 704, 705, 706, 707, 708, 709, 710, 711, 712, 713, 714, 715, 716, 717, 718, 719, 720, 721, 722, 723, 724, 725, 726, 727, 728, 729, 730, 731, 732, 733, 734, 735, 736, 737, 738, 739, 740, 741, 742, 743, 744, 745, 746, 747, 748, 749, 750, 751, 752, 753, 754, 755, 756, 757, 758, 759, 760, 761, 762, 763, 764, 765, 766, 767, 768, 769, 770, 771, 772, 773, 774, 775, 776, 777, 778, 779, 780, 781, 782, 783, 784, 785, 786, 787, 788, 789, 790, 791, 792, 793, 794, 795, 796, 797, 798, 799, ebenfalls besonders vorteilhaft 800%, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 831, 832, 833, 834, 835, 836, 837, 838, 839, 840, 841, 842, 843, 844, 845, 846, 847, 848, 849, 850, 851, 852, 853, 854, 855, 856, 857, 858, 859, 860, 861, 862, 863, 864, 865, 866, 867, 868, 869, 870, 871, 872, 873, 874, 875, 876, 877, 878, 879, 880, 881, 882, 883, 884, 885, 886, 887, 888, 889, 890, 891, 892, 893, 894, 895, 896, 897, 898, 899, ebenfalls besonders vorteilhaft 900%, 901, 902, 903, 904, 905, 906, 907, 908, 909, 910, 911, 912, 913, 914, 915, 916, 917, 918, 919, 920, 921, 922, 923, 924, 925, 926, 927, 928, 929, 930, 931, 932, 933, 934, 935, 936, 937, 938, 939, 940, 941, 942, 943, 944, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 982, 983, 984, 985, 986, 987, 988, 989, 990, 991, 992, 993, 994, 995, 996, 997, 998, 999, oder sogar um 1000 %, oder sogar um 10000% oder sogar um bis zu 100000% oder 1000000% erhöht, wobei jeder Zwischenwert angenommen werden kann. Dies betrifft insbesondere die erhöhte Affinität zu der high affinity site des A-Beta-Monomer oder A-Beta-Oligomer.

Dies wird durch einen entsprechend erniedrigten K_{D}-Wert angezeigt. Der K_{D}-Wert als Maß für die Affinität der Bindung eines modifizierten, insbesondere zyklisierten Peptids an Amyloid-Beta-Monomer oder an A-Beta-Oligomer ist im Vergleich zu einem linearen, bindenden Peptid mit negativer Ladung am freien C-terminus, um 1%, 2, 3, 4, 5, 6, 7, 8, 9, insbesondere 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, insbesondere 99,1, 99,2, 99,3, 99,4, 99,5%, 99,6, 99,7, 99,8, 99,9 bis zu 99,99 oder sogar 99,999% erniedrigt, wobei jeder Zwischenwert angenommen werden kann.

Diese niedrigeren K_{D}-Werte beziehen sich vorteilhaft insbesondere aber nicht ausschließlich auf die high affinity site von A-Beta-Monomer oder A-Beta-Oligomer.

Die modifizierten Peptide können dann effizienter als Sonden für diagnostische Zwecke verwendet werden, als lineare, bindende Peptide mit negativer Ladung am freien C-terminus, insbesondere auch effizienter als ihre linearen Peptid-Pendants mit identischer Aminosäuresequenz verwendet werden.

Sie können aber insbesondere auch effizienter als Therapeutika verwendet werden, als lineare, bindende Peptide mit negativer Ladung am freien C-terminus, insbesondere effizienter als ihre linearen Peptid-Pendants mit identischer Aminosäuresequenz.

Im Rahmen der Erfindung wurde weiterhin erkannt, dass derartig modifizierte Peptide im Vergleich zu Peptiden mit negativer Ladung am freien C-terminus, insbesondere aber im Vergleich zu ihren Peptid-Pendants mit identischer Aminosäuresequenz, zusätzlich auch mit einer höheren Effektivität bzw. Effizienz die Bildung besonders toxischer Amyloid-Beta-Oligomere verhindern oder deren Zerstörung und / oder Endtoxifizierung bewirken. Diese Effektivität ist insbesondere um 1%, 2, 3, 4, 5, 6, 7, 8, 9, insbesondere 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 99,9, besonders vorteilhaft sogar um 100% erhöht.

In einer besonders bevorzugten Ausgestaltung der Erfindung treten die Effekte der gesteigerten Affinität gegenüber A-Beta-Monomer oder A-Beta-Oligomer oder A-Beta-Fibrillen und damit verbundene gleichzeitige Effektivität der Eliminierung, Endtoxifizierung von A-Beta-Oligomeren (bzw. der Bildung) auch in vitro und / oder in vivo auf.

Gegenstand der Erfindung ist auch ein erfindungsgemäßes Peptid zur Bindung an aggregierte A-Beta-Peptide.

Weiterer Gegenstand der Erfindung ist ein Verfahren zur Herstellung des erfindungsgemäßen Peptids durch Peptidsynthese, wie zum Beispiel dem Fachmann bekannt, organische Synthesemethoden für beliebige Verbindungen mit geringem Molekulargewicht (low-molecular weight compounds) und/oder Mutagenese und rekombinante Herstellung.

Die Erfindung betrifft ferner auch eine Zusammensetzung enthaltend die erfindungsgemäßen Peptide, insbesondere zur Behandlung und Diagnose von Morbus Alzheimer.

Gegenstand der vorliegenden Erfindung ist ferner eine Zusammensetzung enthaltend die erfindungsgemäßen Peptide, insbesondere zur Verhinderung von toxischen A-Beta-Oligomeren, oder zur Zerstörung von daraus gebildeten Polymere oder Fibrillen.

Die erfindungsgemäße "Zusammensetzung" kann z. B. ein Impfstoff, ein Medikament (z. B. in Tablettenform), eine Injektionslösung, ein Nahrungs- oder Nahrungsergänzungsmittel sein, enthaltend die erfindungsgemäßen Peptide in einer aufgrund des Fachwissens herzustellenden Formulierung.

Die Erfindung betrifft ferner auch ein KIT enthaltend die erfindungsgemäßen Peptide.

In einem solchen KIT können die erfindungsgemäßen Peptide in Behältern ggf. mit/in Puffern oder Lösungen verpackt sein. Alle Komponenten des KITs können in demselben Behälter oder getrennt voneinander verpackt sein. Ferner kann das KIT Anweisungen für dessen Gebrauch enthalten. Ein solches KIT kann beispielsweise die erfindungsgemäßen in einer Injektionsflasche mit Stopfen und/oder Septum enthalten. Ferner kann darin beispielsweise auch eine Einmal-Spritze enthalten sein.

Ein weiterer Gegenstand der vorliegenden Erfindung ist die Verwendung des erfindungsgemäßen Peptids in-vitro als Sonde zur Identifizierung, qualitativen und/oder quantitativen Bestimmung von Amyloid-Beta-Monomeren Amyloid-Beta-Oligomeren oder A-Beta-Fibrillen.

Gegenstand der vorliegenden Erfindung ist auch eine Sonde, enthaltend die erfindungsgemäßen Peptide zur Identifizierung, qualitativen und/oder quantitativen Bestimmung von Amyloid-Beta-Monomeren oder Amyloid-Beta-Oligomeren.

Solche Sonden sind von großer Bedeutung, da damit eine frühe Diagnose der Alzheimerschen Demenz möglich wird. Mit der Frühdiagnose kann der Krankheit schon in einem sehr frühen Stadium entgegengewirkt werden.

Solche molekularen Sonden enthalten die erfindungsgemäßen Polymere und gegebenenfalls Farbstoffe, Fluoreszenzfarbstoffe, radioaktive Isotope, (PET etc.), Gadolinium (MRI), sowie alternative Stoffe geeignet für die Bildgebung der Sonden und können den Patienten z.B. intravenös injiziert werden. Nach Passage über die Bluthirnschranke können die Sonden an A-Beta-Monomere, A-Beta-Oligomere und/oder Plaques binden. Die so markierten A-Beta-Oligomere und/oder Plaques können mittels bildgebender Verfahren wie z.B. SPECT, PET, CT, MRT, Protonen-MR-Spektroskopie usw. sichtbar gemacht werden.

Ferner betrifft die Erfindung auch die Verwendung des Peptids in-vitro zur Verhinderung von Amyloid-Beta-Oligomeren und/oder Amyloid-Beta-Peptid-Aggregaten und/oder Amyloid-Beta-Fibrillen.

Das erfindungsgemäße Peptid wird auch zur Enttoxifizierung von toxischen Amyloid-Beta-Oligomeren und/oder Aggregaten verwendet. Insbesondere wird es verwendet, um an Amyloid-Beta-Oligomere und/oder Aggregate zu binden und so amorphe, nicht toxische Aggregate zu bilden.

Es wurde erkannt, dass wenn bereits Aβ-Oligomere vorhanden sind, es das Ziel einer Behandlung sein muss, diese durch Substanzen zu adressieren, die eine möglichst hohe Affinität zu A-Beta-Monomer besitzen. De facto kann die Affinität gegenüber A-Beta-Monomer gar nicht groß genug sein und die entsprechende Dissoziationskonstante des erfindungsgemäßen Peptids liegt dann im sub-µM-Bereich, bzw. pM-Bereich oder noch tiefer.

Es wurde im Rahmen der Erfindung erkannt, dass A-Beta-Monomere, als Bausteine der A-Beta-Oligomere, ständig im menschlichen Körper entstehen und vermutlich per se nicht toxisch sind. Es besteht sogar die Möglichkeit, dass Monomere eine positive Funktion besitzen. A-Beta-Monomere können sich in Abhängigkeit von ihrer Konzentration zufällig zusammen lagern. Die Konzentration ist abhängig von ihrer Bildungsund Abbaurate im Körper. Findet mit zunehmendem Alter eine Erhöhung der Konzentration an A-Beta-Monomeren im Körper statt, ist eine spontane Zusammenlagerung der Monomere zu A-Beta-Oligomeren immer wahrscheinlicher. Die so entstandenen A-Beta-Oligomere könnten sich analog zu den Prionen vermehren und letztendlich zur Morbus Alzheimer-Krankheit führen.

Es wurde ferner erkannt, dass ein wichtiger Unterschied zwischen einer Prävention und einer Behandlung oder gar einer Heilung der Alzheimerschen Demenz in der Tatsache begründet liegt, dass eine Prävention möglicherweise schon durch die Verhinderung der Bildung der ersten A-Beta-Oligomere erreicht werden kann. Hierzu sind einige, wenige hochaffine A-Beta-Monomer Liganden ausreichend, die gleichzeitig wenig affin und selektiv (bzw. spezifisch) bezüglich der A-Beta-Oligomere sind.

Diese Anforderungen in Hinblick auf Diagnose (Sonden) und auf die Therapie der Alzheimerschen Demenz sind mit der Bereitstellung der erfindungsgemäßen Peptide erfüllt. Die erfindungsgemäßen Peptide binden im Sinne der Diagnose und/oder Therapie die A-Beta-Monomere oder A-Beta Oligomere mit entsprechend niedriger Dissoziationskonstante. Weiterer Gegenstand ist entsprechend die Verwendung der erfindungsgemäßen Peptide als Therapeutikum von Morbus Alzheimer.

Eine besonders starke Bindung der erfindungsgemäßen Peptide an die Zielmoleküle wird durch eine hohe Spezifität und/oder Affinität zu dem Zielmolekül der erfindungsgemäßen Peptide hervorgerufen. Die gebildeten Komplexe haben eine geringe Dissoziationskonstante (K_{D}-Wert).

Mittels des Thioflavin-T-Tests konnte gezeigt werden, dass die erfindungsgemäßen Peptide die Fibrillenbildung von A-Beta-Peptiden sehr effizient hemmen.

Weiterer Gegenstand ist die Verwendung der erfindungsgemäßen Peptide in einem Verfahren zur Behandlung (in vitro, ex vivo) von Blut, Blutprodukten und/oder Organen, dadurch gekennzeichnet, dass das Blut, die Blutprodukte und/oder Organe dem menschlichen oder tierischen Körper entnommen werden und A-(Amyloid)-Beta-Oligomere entfernt und/oder enttoxifiziert werden.

Der Begriff Bindungsaffinität ist im Sinne der Erfindung das Maß für die Bindungsstärke zwischen den Bindungspartnern bei Protein-Ligand-Wechselwirkungen und die Bindungsspezifität gegenüber A-Beta-Monomer zeigt an, dass eine Bindung nur an A-Beta-Monomere, und keine oder aber nur eine schwache Bindung an andere A-Beta Spezies vorliegt. Als spezifischen Wert wird der Quotient des Bindungssignals von A-Beta-Monomeren zu A-Beta-Oligomeren oder A-Beta-Monomeren zu A-Beta-Fibrillen oder äquivalent für A-Beta-Oligomere angegeben. Spezifische Bindung bedeutet, dass der Quotient größer als 1 ist.

Analog hierzu bedeutet Bindungsspezifität gegenüber A-Beta-Oligomer, dass eine Bindung nur an A-Beta-Oligomere, und keine oder aber nur eine schwache Bindung an andere A-Beta Spezies vorliegt. Als spezifischen Wert wird der Quotient des Bindungssignals von A-Beta-Oligomeren zu A-Beta-Monomer oder A-Beta-Oligomeren zu A-Beta-Fibrillen oder äquivalent für A-Beta-Monomere angegeben. Spezifische Bindung bedeutet wiederum, dass der Quotient größer als 1 ist.

Sind bereits A-Beta-Oligomere vorhanden, sollte es das Ziel einer Behandlung sein, diese durch Substanzen zu adressieren, die eine möglichst hohe Affinität und Spezifität zu A-Beta-Monomeren besitzen, um A-Beta-Oligomere zu eliminieren. Dies gelingt mit den erfindungsgemäßen Peptiden.

Durch die Optimierung des bereits bekannten Peptides D3 soll der Wirkmechanismus ähnlich bzw. identisch bleiben, aber die Substanzen sollen effizientere Eigenschaften besitzen. Die Eigenschaften sind unter anderem Bindungsaffinität und -spezifität für A-Beta-Monomere oder -Oligomere, die Inhibition von A-Beta-Fibrillenbildung, die Inhibierung der A-Beta-Zytotoxizität, die Präzipitation von A-Beta-Oligomeren, die Umwandlung von A-Beta-Fibrillen in nicht-toxische, nicht-amyloide Spezies.

Die Optimierung wurde durch den Austausch von 4, 5, 6 oder 7 Aminosäuren vom ursprünglichen D3 Peptid und einer Deletion erreicht.

Mit Hilfe von Peptid Microarrays wurde D3 optimiert. Dazu wurde in einem ersten Schritt jede Aminosäure des 12mer Peptids gegen 19 natürliche Aminosäuren und 13 weiteren Aminosäuren in der D-enantiomeren Form ausgetauscht. In einem zweiten Schritt wurden die erfolgversprechendsten Austausche miteinander kombiniert. Dabei wurden 4, 5, 6 oder 7 Aminosäuren vom ursprünglichen D3 Peptide ausgetauscht. Des Weiteren wurde 1 Aminosäure deletiert. Da die Bindung der Peptide spezifisch gegen eine Spezies von A-Beta (Monomere, Oligomere oder Fibrillen) gerichtet sein soll, wurden die Microarrays mit den verschiedenen Spezies behandelt und rechnerisch eine Bindung von anderen Spezies, die eventuell im Inkubationsansatz vorhanden sind, ausgeschlossen.

Es wurden 11 Peptide ermittelt, die spezifisch und hochaffin gegen A-Beta-Monomer binden. Dies sind die mit ANK1-7 sowie ANK 15-18 bezeichneten Peptide gemäß der SEQ ID NO: 1-11. Hochaffin heißt dabei mit K_{D}-Werten von 2-fach bis 10-fach niedriger als D3.

| D3 (Stand der Technik): | rprtrlhthrnr |
|---|---|
| a) "ANK1" (freier N-terminus, amidierter C-terminus): | rkrirlvyhinr |
| b) "ANK2" (freier N-terminus, amidierter C-terminus): | rkrirl06yhinr |
| c) "ANK3" (freier N-terminus, amidierter C-terminus): | rkrirl06yhwnr |
| d) "ANK4" (freier N-terminus, amidierter C-terminus): | rkrirlvyhwnr |
| e) "ANK5" (freier N-terminus, amidierter C-terminus): | rkrvrlvyhkkr |
| f) "ANK6" (freier N-terminus, amidierter C-terminus): | rkrirlvtkkkr |
| g) "ANK7" (freier N-terminus, amidierter C-terminus): | rkrvrl02thikr |
| h) "ANK15" (freier N-terminus, amidierter C-terminus): | rprvrl06yhwnr |
| i) "ANK16" (freier N-terminus, amidierter C-terminus): | rkr07rlvtkrnr |
| j) 'ANK17" (freier N-terminus, amidierter C-terminus): | rkrirl06yhikr |
| k) "ANK18" (freier N-terminus, amidierter C-terminus): | rpr07rlhtkkkr |

mit
02: 4-Fluoro-phenylalanin (D)
06: Phenylglycin (D)
07: D-Homoarginin

Alle Peptide werden erfindungsgemäß auch als Doppelpeptide und/oder in zyklisierter Form genutzt. Dadurch kann die Effektivität der Peptide weiter gesteigert werden.

Die Peptide ANK1-7 sowie ANK15-18 können durch die Bindung an A-Beta-Monomer als Medikament gegen die Alzheimersche Demenz genutzt werden. Oligomer spezifische Peptide können auf Grund ihrer spezifischen Bindung an A-Beta-Oligomere zusätzlich als Sonden zur Diagnose genutzt werden.

### Ausführungsbeispiele

Im Weiteren wird die Erfindung an Hand von Ausführungsbeispielen und der beigefügten Figuren näher erläutert, ohne dass es hierdurch zu einer Beschränkung der Erfindung kommen soll.

Es zeigen:
- Figur 1:: a) Bindungssignale der erfindungsgemäßen Peptide an A-Beta-Monomere, A-Beta-Oligomere und A-Beta-Fibrillen.
b) Bindungsspezifität der erfindungsgemäßen Peptide.
- Figur 2:: Reduktion von Amyloid-Beta-Oligomere durch die erfindungsgemäßen Peptide.
- Figur 3:: a) THT-Assay erfindungsgemäßer Peptide,
b) Ausgewerteter THT Assay mit THT Fluoreszenz bezogen auf Amyloid-Beta im Sättigungspunkt.
- Figur 4:: a) MTT-Assay an SH-SY5 Zellen ohne A-Beta Coinkubation.
b) MTT-Assay an SH-SY5 Zellen mit A-Beta Coinkubation.

Für das Peptid Microarray mit D3 Derivaten wurde dieses über den C-Terminus auf Glasobjektträgern kovalent wie nachfolgend dargestellt verknüpft.
∘ 1ste Generation: D3 mit jeweils 1 Austausch gegen 19 natürliche Aminosäuren und gegen 13 unnatürliche Aminosäuren
∘ 2te Generation: Die Austausche, die einen effizientere Bindung der Peptide an A-Beta bewirken, wurden miteinander kombiniert.
   - Vorbehandlung der Peptide Microarray Slides:
      ∘ 3x kurz in Ethanol (96%) tauchen und anschließend mit H₂O waschen (30 min) ∘ kurz trocken zentrifugieren
   - Präparation von Monomeren und Oligomeren:
      ∘ Größenausschlußchromatographie nach Johansson et al. 2006 (Johansson, A.-S. et al. Physiochemical characterization of the Alzheimer's disease-related peptides Aβ1-42Arctic and Aß1-42wt. FEBS Journal 273, 2618-2630 (2006)).
      ∘ Oligomere wurden bei 8-9 ml fraktioniert, Monomere bei 16-17 ml.
   - Präparation von Fibrillen:
      ∘ Inkubation von A-Beta über 3 Tage, Zentrifugieren, Überstand verwerfen und in Puffer resuspendieren (3x-ige Wiederholung)
   - Durchführung Peptid Microarray:
      ∘ Inkubation mit FITC-A-Beta Monomeren, -Oligomeren, -Fibrillen und FITC für 1 h bei Raumtemperatur.
      ∘ Waschen: TBS-T 3 x kurz (nur TBS-T-Puffer gewechselt), 3x 10 min in einem Falcon, 3x H₂O, 3x 10 min
      ∘ Trocknen durch Zentrifugation
   - Die Bindung des Fluoreszenz gelabelten A-Beta mit den immobilisierten Peptiden wird über den Fluoreszenzfarbstoff mit dem Microarray Reader FLA800 von Fuji Film detektiert. Je mehr A-Beta an dem Peptid bindet, umso höher ist das Fluoreszenzsignal.
∘ Carrier Set Mode: Slides
∘ Resolution: 10 µm
∘ Scan Mode: High Sensitivity (Slow): Scan pixel at 200 mm/s
∘ Laser: 473 nm
∘ Filter: 530 DF 20
∘ Photo-multiplier tube (PMT) HV (%): 100
∘ Bilder wurden als .tif-Dateien gespeichert

Die Auswertung der Fluoreszenzsignale erfolgt mit der Software AIDA Metrix von Raytest:

| Grid Definition 1^{st} Level | |
|---|---|
| Rows | 1 |
| Columns | 1 |
| 2^{nd} level | |
| Rows | 12 |
| Columns | 4 |
| X Spacing | 0 |
| Y Spacing | 0 |
| 3^{rd} level | |
| Rows | 9 |
| Columns | 9 |
| Diameter | 80 µm |
| Backround definition | |
| Local Dot Rings | |
| Inflate Dots | 150 µm |
| Ring width | 30 µm |
| By area | mean |
| Aligment defaults (Parameter) | |
| Diameter | 100 % |
| Mobility | Restricted |
| | Align grid, align dots |
| Aligment defaults (Process) | |
| Bkg deviation | |
| Threshold | 2.0*StdDev |

Um die Reproduzierbarkeit der Ergebnisse zu gewährleisten, wird die Inkubation der Peptid Microarrays je Spezies mindestens 3 x durchgeführt.

Die gemessenen Fluoreszenzsignale der Reproduktionen werden normiert mit einer Median-basierten Normierung und die Kontrolle wurde vom Signal abgezogen. Damit wurde verhindert, dass die Bindung des Fluoreszenz-gelabelten A-Beta über den Farbstoff stattfindet.

Die Bindungssignale von einer A-Beta Spezies an ein Peptid wurde gemittelt.

Für die Bestimmung der Bindungsspezifität wird der Quotient von der Spezies, zu dem das Peptide spezifisch binden soll, zu einer anderen Spezies gebildet.

Beispiel: Bindungsspezifität des Peptides X zu A-Beta Monomeren = Bindungssignal von Peptide X an Monomere/ Bindungssignal von Peptide X an Oligomere.

Die erfindungsgemäßen Peptide ANK1-7 sowie ANK 15-18 führen zu einer Inhibition der A-Beta-Fibrilisierung, Inhibition der A-Beta-Zelltoxizität, Eliminierung von A-Beta-Oligomeren sowie zu einer Umwandlung von A-Beta-Fibrillen in nicht-toxische, nicht-amyloide Spezies. Die Bindungsaffinität und Spezifität zu A-Beta-Monomeren ist dabei gegeben.

Die Bindungsaffinität ist dabei das Maß für die Bindungsstärke zwischen den Bindungspartnern bei Protein-Ligand-Wechselwirkungen und die Bindungsspezifität zeigt an, dass eine Bindung nur an A-Beta-Monomere, und keine oder aber nur eine schwache Bindung an andere A-Beta Spezies vorliegt. Als spezifischen Wert wird hier der Quotient des Bindungssignals von A-Beta-Monomeren zu A-Beta-Oligomeren oder A-Beta-Monomeren zu A-Beta-Fibrillen oder äquivalent für A-Beta-Oligomere angegeben. Spezifische Bindung bedeutet, dass der Quotient größer als 1 ist.

Im Detail wurden folgende Daten ermittelt:

| | Bindungssignal an A-Beta Monomere | Bindungssignal an A-Beta Oligomere | Bindungssignal an A-Beta Fibrillen | Bindungsspezifität Monomer zu Oligomer | Bindungsspezifität Monomer zu Fibrillen |
|---|---|---|---|---|---|
| ANK1 | 6.6 | 1.9 | 1.3 | 3.5 | 5.1 |
| ANK2 | 5.6 | 1.6 | 0.8 | 3.4 | 7.1 |
| ANK3 | 3.6 | 0.6 | 0.2 | 6.5 | 21.9 |
| ANK4 | 4.4 | 1.3 | 1.0 | 3.3 | 4.3 |
| ANK5 | 4.3 | 0.7 | 2.0 | 5.9 | 2.2 |
| ANK6 | 4.9 | 1.2 | 2.5 | 4.1 | 1.9 |
| ANK7 | 2.1 | 0.3 | 0.3 | 6.4 | 6.8 |
| ANK15 | 1.3 | 0.6 | 0.1 | 2.2 | 12.2 |
| ANK16 | 11.9 | 1.4 | 1.5 | 8.6 | 8.1 |
| ANK1 7 | 4.7 | 1.0 | 1.9 | 4.8 | 2.5 |
| ANK18 | 1.2 | 0.2 | 0.1 | 7.0 | 9.4 |
| D3 | 0.5 | 0.6 | 0.8 | 0.8 | 0.6 |

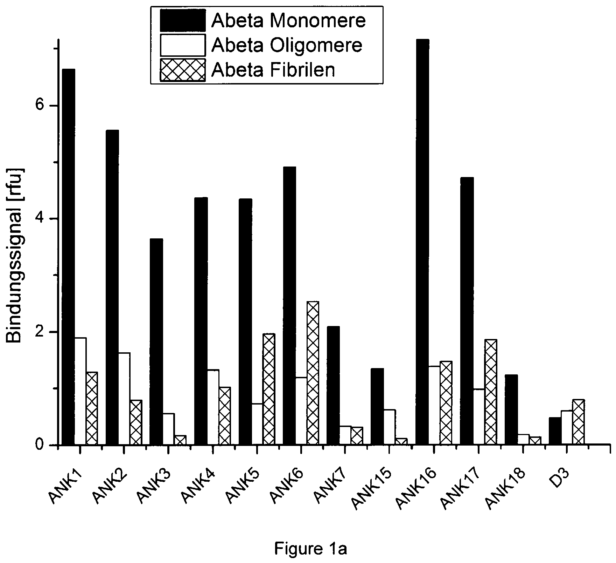

Figur 1a zeigt die Bindungssignale für ANK1-ANK7 und ANK15-18 im Vergleich zu D3 an A-Beta Monomere, -Oligomere und -Fibrillen. Die Peptide binden an A-Beta-Monomere wesentlich effizienter als an A-Beta-Oligomere und an A-Beta-Fibrillen, was durch einen deutlichen Unterschied in dem Bindungssignal erkennbar ist. Deutlicher Unterschied heißt mindestens eine Differenz von 1,5 rfu zwischen dem Bindungssignal von A-Beta-Monomere und A-Beta-Oligomere und/oder A-Beta-Fibrillen.

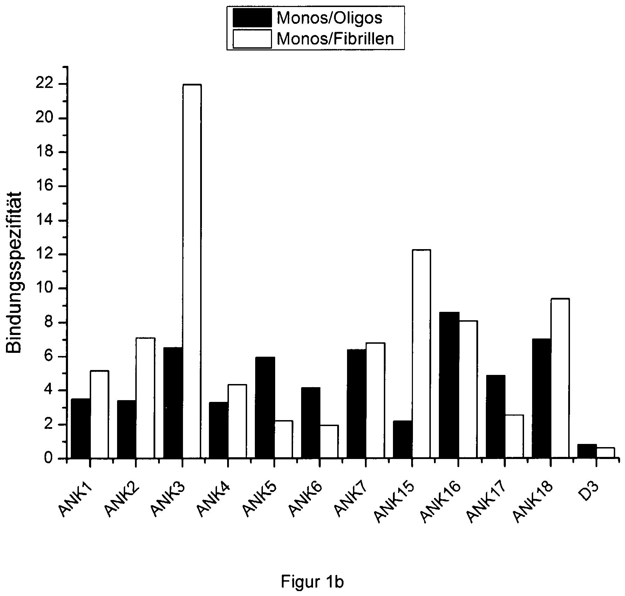

Figur 1b zeigt die Bindungsspezifität für ANK1-7 und ANK 15-18. Es wird deutlich, dass bei allen erfindungsgemäßen Peptiden die Bindungsspezifität gegenüber A-Beta-Monomer größer ist als 1. D3 besitzt hingegen eine Bindungsspezifität gegenüber A-Beta-Monomer von unter 1.

Mittels des QIAD-Verfahrens zur quantitativen Charakterisierung amyloider Peptide und/oder Proteine in einer Probe konnten die Eigenschaften der Substanzen quantifiziert werden. Vorinkubiertes A-Beta wurde mit den Peptiden coinkubiert. Mit Hilfe einer Dichtegradientenzentrifugation wurden die verschiedenen A-Beta Spezies getrennt und mittels RP-HPLC Abeta in den einzelnen Fraktionen quantifiziert. Es wurde die Eliminierung der A-Beta-Oligomere in Fraktion 4-6 analysiert.

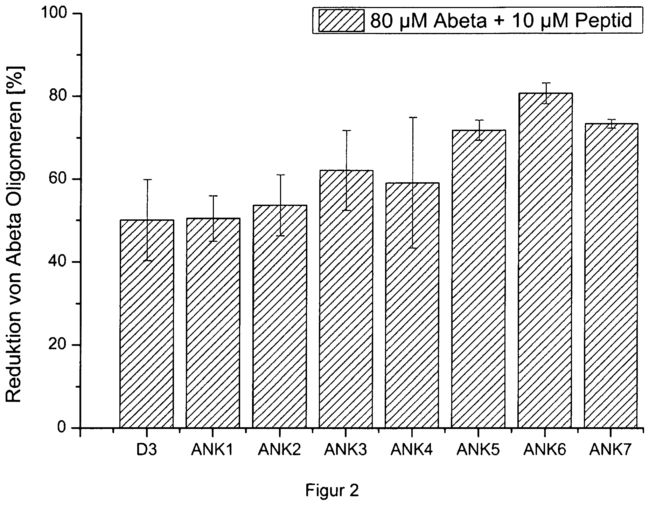

Es wird gezeigt, dass insbesondere ANK5, ANK6 und ANK7 A-Beta-Oligomere effizienter als D3 eliminieren, siehe die Figur 2. Zum einen kann die spezifische Bindung an Monomere, die Bildung von Oligomeren verhindern, ohne dabei zu einer Reduktion der Konzentration an A-Beta-Monomer zu führen und zum anderen können bereits entstandene A-Beta-Oligomere durch eine Behandlung mit einem A-Beta-Monomer stabilisierenden Wirkstoff aus dem dynamischen Gleichgewicht der verschiedenen Aggregatspezies entsprechend des Prinzips von Le Chatelier entfernt werden und damit bewirken, dass A-Beta-Oligomere zu A-Beta-Monomeren abgebaut werden.

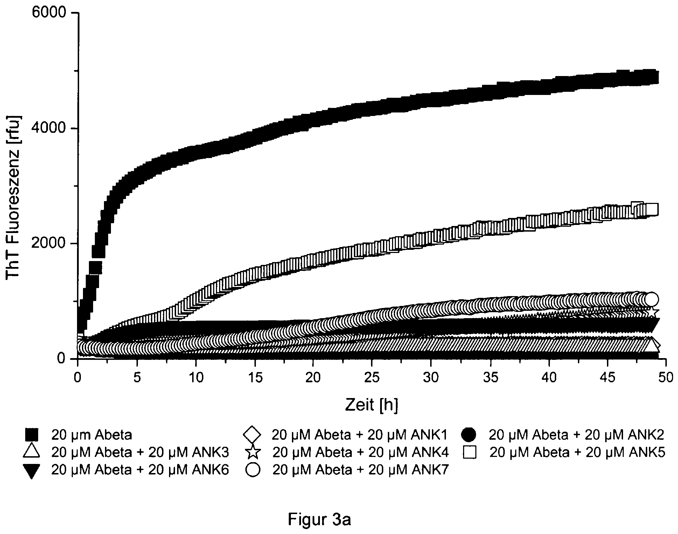

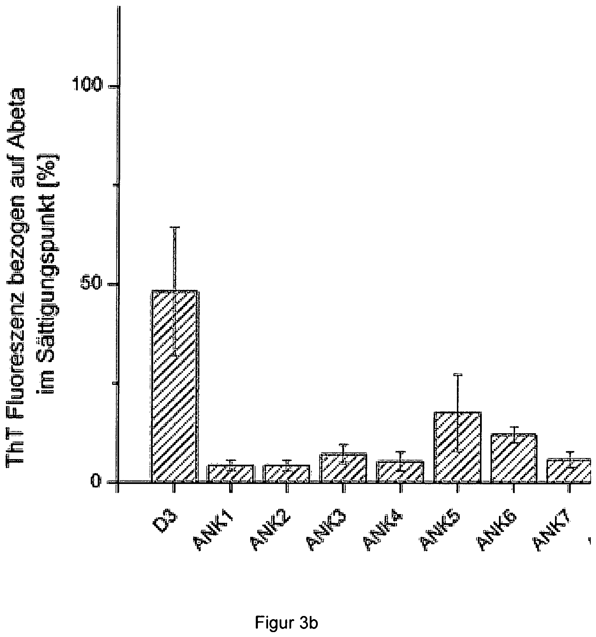

Im ThT-Assay zeigt sich eine deutliche Reduktion der ThT-positiven A-Beta-Fibrillen nach einer Co-Inkubation mit den getesteten Substanzen (Figuren 3a und 3b). Dies deutet darauf hin, dass die Aggregation inhibiert wird oder amorphe Aggregate gebildet werden.

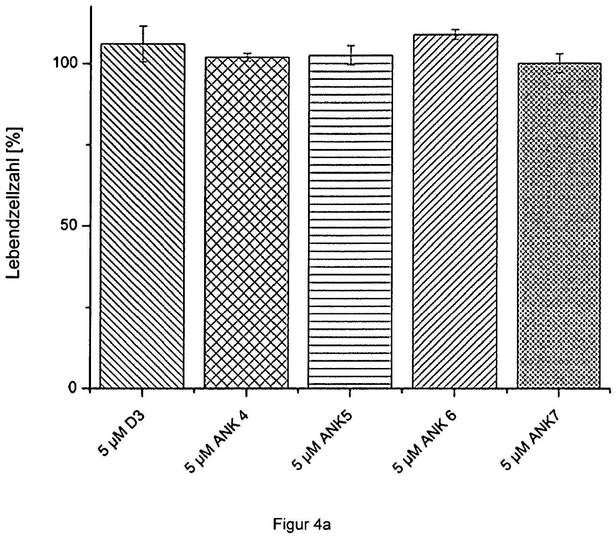

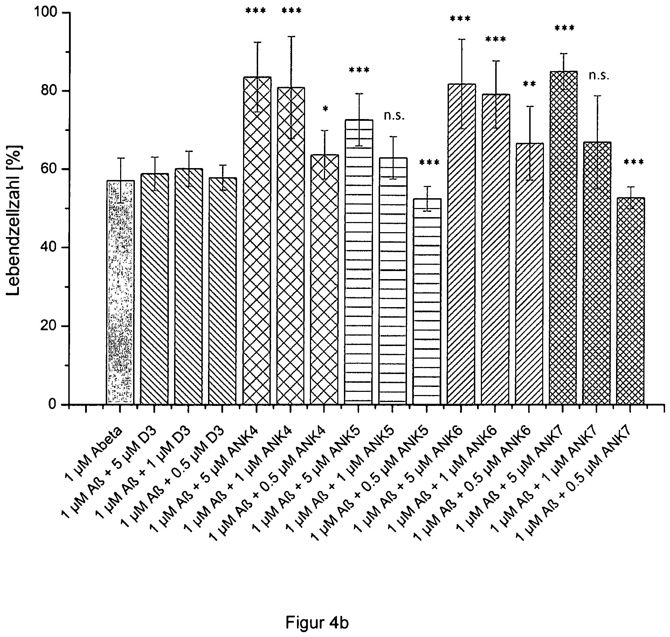

Es konnte in therapeutisch relevanten Konzentrationen keine nennenswerte Toxizität der erfindungsgemäßen Peptide in dem, dem Fachmann bekannten, MTT-Assay an SH-SY5Y-Zellen festgestellt werden (Figur 4). Dagegen konnte gezeigt werden, dass die durch A-Beta hervorgerufene Toxizität durch die Substanzen ANK4, ANK5, ANK6 und ANK7 reduziert wird. A-Beta-Oligomer ist nach dem aktuellen Wissenstand die toxische Spezies. Da mit den Peptiden die A-Beta-Oligomere eliminiert werden, wird auch die A-Beta-induzierte Toxizität reduziert.

Im Detail:
Figur 2 zeigt, dass bei einem Verhältnis von A-Beta zu Peptid von 8:1 die Peptide ANK5, ANK6 und ANK7 A-Beta-Oligomere signifikant effizienter reduzieren als D3. Die anderen Peptide sind in dieser Konzentration vergleichbar effizient wie D3. D3 eliminiert die A-Beta-Oligomere um 50 ± 10 %, ANK1 um 51 ± 6 %, ANK2 um 54 ± 7 %, ANK3 um 62 ± 10 %, ANK4 um 59 ± 16 %, ANK5 um 72 ± 2 %, ANK6 um 81 ± 3 % und ANK7 um 73 ± 1 %.

Erfindungsgemäße Peptide zeigen somit eine im Vergleich zu D3 um einen Faktor von bis zu 1,6 erhöhte Reduktion an A-Beta-Oligomeren.

Figur 3 zeigt, dass ANK1-7 deutlich die Bildung von ThT-positiven A-Beta-Fibrillen reduziert. Die ThT-Fluoreszenz bleibt während des gesamten Messzeitraums (48 h) für die Peptide ANK1, ANK2 und ANK3 auf dem Niveau des ersten Messwertes, für die Peptide ANK4, ANK6 und ANK7 ist hingegen ein leichter Anstieg zu erkennen (entspricht 13 - 21 % der ThT Fluoreszenz von Abeta nach 48 h) und für ANK5 ein Anstieg auf 1029 rfu, was 53 % der ThT Fluoreszenz von der A-Beta Kontrolle nach 48 h entspricht.

Figur 4a zeigt, dass ANK4, ANK5, ANK6 und ANK7 alleine keinen toxischen Effekt auf die Zellen aufweist. Mit einer Coinkubation von A-Beta mit ANK4, ANK5, ANK6 und ANK7 in verschiedenen Konzentrationen konnte eine Reduktion der A-Beta-Toxizität erreicht werden, siehe Figur 4b. Diese Reduktion ist mit den Substanzen ANK4 und ANK6 effizienter als mit D3. Die Signifikanzen sind bezogen auf die jeweilige Konzentration mit D3 (* p ≤ 0.05; ** p ≤ 0.01; *** p ≤ 0.001; n. s. nicht signifikant).

## Patentansprüche

1. Spezifisch eine Amyloid-Beta-Spezies bindendes Peptid, das im Wesentlichen aus D-Aminosäuren besteht, enthaltend mindestens eine Aminosäuresequenz ausgewählt aus der Gruppe bestehend aus SEQ ID NO: 1, SEQ ID NO: 2, SEQ ID NO: 3, SEQ ID NO: 4, 'SEQ ID NO: 5, SEQ ID NO: 6, SEQ ID NO: 7, SEQ ID NO: 8, SEQ ID NO: 9, SEQ ID NO. 10 und/oder SEQ ID NO. 11 und deren Homologe mit einer Identität der Aminosäuresequenz von mindestens 80%, wobei die Homologe einen Kd-Wert von 2-fach bis 10-fach niedriger als das Peptid mit der Aminosäuresequenz rprtrlhthrnr aufweisen,
sowie Polymere der SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO. 10, und/oder SEQ ID NO. 11 und deren Homologe, mit einer Identität der Aminosäuresequenz von mindestens 80%, wobei die Homologe einen Kd-Wert von 2-fach bis 10-fach niedriger als das Peptid mit der Aminosäuresequenz rprtrlhthrnr aufweisen.

2. Peptid nach Anspruch 1 zur Anwendung in der Medizin.

3. Peptid nach Anspruch 1 zur Anwendung bei der Behandlung der Morbus Alzheimer.

4. Peptid nach Anspruch 1 zur Anwendung bei der Diagnose der 'Morbus Alzheimer in Patienten.

5. Peptid zur Anwendung nach einem der vorangehenden Ansprüche 2 bis 3 zur Hemmung der Bildung von A-Beta-Fibrillen.

6. Peptid zur Anwendung nach einem der vorangehenden Ansprüche 2 bis 3 zur Bindung an aggregierte Amyloid-Beta-Spezies.

7. KIT, enthaltend ein Peptid nach Anspruch 1 oder zur Verwendung gemäß einem der Ansprüche 2 bis 6.

8. Zusammensetzung, enthaltend ein Peptid nach Anspruch 1.

9. Verwendung eines Peptids nach Anspruch 1 in-vitro als Sonde zur spezifischen Identifizierung, quantitativen und/oder qualitativen Bestimmung von Amyloid-Beta-Monomeren, Amyloid-Beta-Fibrillen und/oder Amyloid-Beta-Oligomeren.

10. Verwendung eines Peptids nach Anspruch 1 in-vitro zur Verhinderung von Amyloid-Beta-Oligomeren und/oder Amyloid-Beta-Peptidaggregaten.

11. Verwendung eines Peptids nach Anspruch 1 in-vitro zur Enttoxifizierung von toxischen Amyloid-Beta-Oligomeren und/oder Aggregaten.

12. Verwendung nach Anspruch 11 **dadurch gekennzeichnet, dass** Amyloid-Beta-Oligomere und/oder Aggregate mit dem Peptid nach Anspruch 1 amorphe, nicht toxische Aggregate bilden.

13. Verwendung von mindestens einer Aminosäuresequenz, die im Wesentlichen aus D-Aminosäuren besteht, ausgewählt aus der Gruppe bestehend aus SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID 15 NO: 9, SEQ ID NO. 10 und/oder SEQ ID NO. 11 und deren Homologe mit einer Identität der Aminosäuresequenz von mindestens 80%, wobei die Homologe einen Kd-Wert von 2-fach bis 10-fach niedriger als das Peptid mit der Aminosäuresequenz rprtrlhthrnr aufweisen,
sowie Polymere der SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO. 10, und/oder SE. Q ID NO. 11 und deren Homologe, mit einer Identität der Aminosäuresequenz von mindestens 80%, wobei die Homologe einen Kd-Wert von 2-fach bis 10-fach niedriger als das Peptid mit der Aminosäuresequenz rprtrlhthrnr aufweisen,
zur spezifischen Bindung in-vitro an A-Beta-Monomer.

14. Peptid nach Anspruch 1 zur Anwendung bei der Prävention von Morbus Alzheimer.

## Claims

1. Specifically to an amyloid-beta-species binding peptide, consisting essentially of D-amino acids, containing at least one amino acid sequence selected from the group consisting of SEQ ID NO: 1, SEQ ID NO: 2, SEQ ID NO: 3, SEQ ID NO: 4, 'SEQ ID NO: 5, SEQ ID NO: 6, SEQ ID NO: 7, SEQ ID NO: 8, SEQ ID NO: 9, SEQ ID NO. 10 und/oder SEQ ID NO. 11 and their homologues with an identity of the amino acid sequence of at least 80%, the homologues having a Kd-value of from twice up to tenfold lower than the peptide with the amino acid sequence rprtrlhthrnr,
and polymers of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO. 10, und/oder SEQ ID NO. 11 and their homologues , with an identity of the amino acid sequence of at least 80%, the homologues having a Kd-value of from twice up to tenfold lower than the peptide with the amino acid sequence rprtrlhthrnr.

2. Peptide according to claim 1 for the application in medicine.

3. Peptide according to claim 1 for the application in the treatment of Alzheimer's disease.

4. Peptide according to claim1 for the application in the diagnosis of Alzheimer's disease.

5. Peptide for the application according to any of the preceding claims 2 to 3 for the inhibition of the formation of a-beta-fibrils.

6. Peptide for the application according to any of the preceding claims 2-3 for the binding to aggregated amyloid-beta-species.

7. KIT, containing a peptide according to claim 1 or for the use according to any one of claims 2 to 6.

8. Composition, comprising a peptide according to claim 1.

9. Use of a peptide according to claim 1 in vitro as a probe for the specific identification, quantitative and/or qualitative determination of amyloid-beta monomers, amyloid-beta fibrils and/or amyloid-beta oligomers.

10. Use of a peptide according to claim 1 in vitro for the inhibition of amyloid-beta oligomers and/or amyloid-beta peptide aggregates.

11. Use of a peptide according to claim 1 in vitro for the de-toxification of toxic amyloid-beta oligomers and/or aggregates.

12. Use according to claim 11 , **characterised in that** amyloid-beta-oligomers und/ oder aggregates with the peptide according to claim 1 form amorphous, non-toxic aggregates.

13. Use of at least one amino acid sequence which consists essentially of D-amino acids, selected from the group consisting of SEQ ID NO: 1, SEQ ID NO: 2, SEQ ID NO: 3, SEQ ID NO: 4, 'SEQ ID NO: 5, SEQ ID NO: 6, SEQ ID NO: 7, SEQ ID NO: 8, SEQ ID NO: 9, SEQ ID NO. 10 und/oder SEQ ID NO. 11 and their homologues with an identity of the amino acid sequence of at least 80%, the homologues having a Kd-value of from twice up to tenfold lower than the peptide with the amino acid sequence rprtrlhthrnr,
and polymers of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO. 10, und/oder SEQ ID NO. 11 and their homologues, with an identity of the amino acid sequence of at least 80%, wherein the homologues have a Kd-value of from twice up to tenfold lower than the peptide with the amino acid sequence rprtrlhthrnr
for the specific binding in vitro to A-beta monomers.

14. Peptide according to claim 1 for the application in the prevention of Alzheimer's disease.

## Revendications

1. Un peptide se liant spécifiquement à une espèce bêta-amyloïde, consistant essentiellement des D-acides aminés, contenant au moins une séquence d'acide aminé choisie du groupe consistant de SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 ,SEQ ID NO: 10 et/ou SEQ ID NO. 11. et de leurs homologues avec une identité de la séquence d'acide aminé d'au moins 80%, les homologues ayant un valeur Kd-de deux fois jusqu'à dix fois plus bas que le peptide avec la séquence d'acide aminé rprtrlhthrnr,
et des polymères de SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 ,SEQ ID NO: 10 et/ou SEQ ID NO. 11. et de leurs homologues avec une identité de la séquence d'acide aminé d'au moins 80%, les homologues ayant un valeur Kd de deux fois jusqu'à dix fois plus bas que le peptide avec la séquence d'acide aminé rprtrihthrnr.

2. Le peptide selon la revendication 1 pour l'application en la médecine.

3. Le peptide selon la revendication 1 pour l'application en le traitement de la maladie d'Alzheimer.

4. Le peptide selon la revendication 1 pour l'application en le diagnostic de la maladie d'Alzheimer.

5. Le peptide pour l'application selon l'une quelconque des revendications précédentes 2 à 3 pour inhiber la formation des fibrilles bêta-amyloïde.

6. Le peptide pour l'application selon l'une quelconque des revendications précédentes 2 à 3 pour la liaison aux espèces bêta-amyloïde agrégées.

7. KIT, contenant un peptide selon la revendication 1 ou pour l'utilisation selon l'une quelconque des revendications 2 à 6.

8. Une composition, contenant un peptide selon la revendication 1.

9. L'utilisation d'un peptide selon la revendication 1 in vitro comme une sonde pour l'identification spécifique, la détermination quantitative et/ou qualitative des fibrilles bêta-amyloïde et/ou des oligomères bêta-amyloïde.

10. L'utilisation d'un peptide selon la revendication 1 in vitro pour la prévention des oligomères bêta-amyloïdes et/ou des agrégats de peptides bêta -amyloïde.

11. L'utilisation d'un peptide selon la revendication 1 in vitro pour la détoxification des oligomères bêta-amyloïde toxiques et/ou des agrégats.

12. L'utilisation selon la revendication 11, **caractérisee en ce que** des oligomères bêta-amyloïde et/ou des agrégats forment avec le peptide selon la revendication 1 des agrégats amorphes, non-toxiques.

13. Utilisation d'au moins d'une séquence d'acide aminé, consistant essentiellement des D-acides aminés, choisies du groupe consistant de SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 , SEQ ID NO: 10 et/ou SEQ ID NO. 11. et de leurs homologues avec une identité de la séquence d'acide aminé d'au moins 80%, les homologues ayant une valeur Kd-de deux fois jusqu'à dix fois plus bas que le peptide avec la séquence d'acide aminé rprtrlhthrnr et
des polymères de SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 ,SEQ ID NO: 10 et/ou SEQ ID NO. 11. et de leurs homologues avec une identité de la séquence d'acide aminé d'au moins 80%, les homologues ayant un valeur Kd de deux fois jusqu 'à dix fois plus bas que le peptide avec la séquence d'acide aminé rprtrlhthrnr.
pour la liaison spécifique in vitro au monomère A-bêta.

14. Le peptide selon la revendication 1 pour l'application en la prévention de la maladie d' Alzheimer.


### SEQUENCE LISTING

<110> Forschungszentrum Juelich GmbH
<120> Spezifisch A-Beta-Spezies bindende Peptide für die Therapie und/oder Diagnose der Alzheimerschen Demenz
<130> PT 1.2695 PCT
<160> 11
<170> PatentIn version 3.5
<210> 1
   <211> 12
   <212> PRT
   <213> Artificial Sequence
<220>
   <223> D-Peptide, ANK6
<400> 1
   Arg Lys Arg Ile Arg Leu Val Thr Lys Lys Lys Arg
   1 5 10
<210> 2
   <211> 12
   <212> PRT
   <213> Artificial Sequence
<220>
   <223> D-Peptide, ANK7 (mit 4-Fluoro-phenylalanin (D) an Position 7 an Stelle
   von Phenylalanin)
<400> 2
   Arg Lys Arg Val Arg Leu Phe Thr His Ile Lys Arg
   1 5 10
<210> 3
   <211> 12
   <212> PRT
   <213> Artificial sequence
<220>
   <223> D-Peptide, ANK5
<400> 3
   Arg Lys Arg Val Arg Leu Val Tyr His Lys Lys Arg
   1 5 10
<210> 4
   <211> 12
   <212> PRT
   <213> Artificial sequence
<220>
   <223> D-Peptide, ANK4
<400> 4
   Arg Lys Arg Ile Arg Leu Val Tyr His Trp Asn Arg
   1 5 10
<210> 5
   <211> 12
   <212> PRT
   <213> Artificial Sequence
<220>
   <223> D-Peptide, ANK1
<400> 5
   Arg Lys Arg Ile Arg Leu Val Tyr His Ile Asn Arg
   1               5                   10
<210> 6
   <211> 12
   <212> PRT
   <213> Artificial sequence
<220>
   <223> D-Peptide, ANK2 (mit Phenylglycin (D) an Position 7 an Stelle von Glycin)
<400> 6
   Arg Lys Arg Ile Arg Leu Gly Tyr His Ile Asn Arg
   1               5                   10
<210> 7
   <211> 12
   <212> PRT
   <213> Artificial sequence
<220>
   <223> D-Peptide, ANK3 (mit Phenylglycin (D) an Position 7 an stelle von Glycin)
<400> 7
   Arg Lys Arg Ile Arg Leu Gly Tyr His Trp Asn Arg
   1               5                   10
<210> 8
   <211> 12
   <212> PRT
   <213> Artificial Sequence
<220>
   <223> D-Peptide, ANK15 (mit Phenylglycin (D) an Position 7 an Stelle von
   Glycin)
<400> 8
   Arg Pro Arg Val Arg Leu Gly Tyr His Trp Asn Arg
   1               5                   10
<210> 9
   <211> 12
   <212> PRT
   <213> Artificial Sequence
<220>
   <223> D-Peptide, ANK16 (mit Homoarginin (D) an Position 4 an stelle von
   Arginin)
<400> 9
   Arg Lys Arg Arg Arg Leu Val Thr Lys Arg Asn Arg
   1 5 10
<210> 10
   <211> 12
   <212> PRT
   <213> Artificial Sequence
<220>
   <223> D-Peptide, ANK17 (mit Phenylglycin (D) an Position 7 an Stelle von
   Glycin)
<400> 10
   Arg Lys Arg Ile Arg Leu Gly Tyr His Ile Lys Arg
   1 5 10
<210> 11
   <211> 12
   <212> PRT
   <213> Artificial sequence
<220>
   <223> D-Peptide, ANK18 (mit Homoarginin (D) an Position 4 an Stelle von
   Arginin)
<400> 11
   Arg Pro Arg Arg Arg Leu His Thr Lys Lys Lys Arg
   1 5 10